# EUROPEAN PATENT APPLICATION

(11) **EP 4 756 033 A1**
(43) Date of publication of application: **10.06.2026**
(21) Application number: 25180723.6
(22) Date of filing: 04.06.2025
(51) Int. Cl.: C12P 17/16, D06P 1/22

(54) **BIOSYNTHESIS OF INDIGO DYESTUFF WITH TUNABLE COLOR HUE**

(30) Priority: 03.12.2024 US 202418966177; 26.05.2025 US 202519218609
(71) Applicant: Nano and Advanced Materials Institute Limited, Shatin, New Territories (HK)
(72) Inventor: O, Wing Nien Wylie, Hong Kong (HK); TAM, Dick Yan, Hong Kong (HK); YAN, GuoZhuangQi, Hong Kong (HK); CHENG, Dan, Hong Kong (HK)
(74) Representative: Dr. Gassner & Partner mbB

(57) **Abstract**

A biosynthetic method and composition for producing an Indigo dyestuff including Indigotin and Indirubin. Tryptophanase (TRP) and flavin-containing monooxygenase (FMO) are generated from a genetically-modified organism having a vector coding for tryptophanase and flavin-containing monooxygenase. The conversion of L-tryptophan to Indigo dyestuff is converted by the generated TRP and FMO in the presence of one or more bioactive additives selected from the cysteine, 2-oxindole, or 2-indoxyl to form a dyestuff having a ratio of Indigotin to Indirubin by weight between 1:0.1 to 1:4. The dyestuff has a color hue defined by CIE XYZ color space with X: 0.1920 to 0.2481, Y: 0.2537 to 0.2019, Z: 0.5543 to 0.5501. Selectable and reproduceable color hues are provided.

## Description

### Reference to Sequence Disclosure:

The sequence listing file under the file name "P3315EP01_Sequence Listing.xml" submitted in ST.26 XML file format with a file size of 5.51 KB created on May 15^{th}, 2025 and filed on May 26^{th}, 2025 is incorporated herein by reference.

### Field of the Invention:

The present invention relates to biosynthesis of Indigo dyestuff and, more particularly, to the biosynthesis of Indigo dyestuff having tunable color hues.

### Background:

Textile dyeing is the second largest cause of global water pollution. With the textile industry's increasing focus on sustainable development, along with stringent government environmental regulations regarding water pollution, the demand for plant-based dyes to replace synthetic dyes is also rising. The plant-based dye market is on the order of five billion US dollars with expected annual increases due to the increasing demand for these dyes.

However, traditional plant-based dyes often result in uneven colors and poor color fastness, making them difficult to apply in fashion clothing. Good color wash-fastness is the most attractive feature that drives constant market growth in fashion clothing. In addition, some products still require the use of toxic chemicals, such as amines, sodium sulfide, and caustics in the dyeing process.

Among plant-based dyes, Indigo dyestuff extracted from plants has an extraction rate of no more than 1%, while the content of Indigotin does not exceed 20%. Indigo dye extracted from the leaves of Polygonum tinctorium can produce beautiful colors on denim. The leaves require at least one year to harvest, however.

Synthetic Indigo dyestuff can also be produced using chemical synthesis from petrochemical precursors. Synthetic Indigo dyestuff production typically begins with aniline, a derivative of benzene that is sourced from petroleum. Aniline is a highly reactive compound, making it an ideal precursor for the synthetic Indigo pathway. The aniline undergoes several chemical reactions, most notably chlorination, to create intermediates such as N-phenylglycine. This intermediate is key in forming the indigo molecule. N-phenylglycine is then subjected to an alkaline fusion process, which promotes its transformation into indoxyl - a precursor to Indigotin. Aniline is also a precursor to isatin in which its oxidative coupling can form Indirubin.

Oxidative coupling to form Indigotin: The indoxyl molecules undergo a final oxidation reaction, typically in the presence of oxygen, to form Indigotin (the blue dye component of Indigo). This oxidation causes two indoxyl molecules to couple and form the characteristic blue color.

However, synthetic Indigo production involves several hazardous intermediates and byproducts, such as formaldehyde, cyanide compounds, and aniline derivatives, which are toxic and must be carefully managed to minimize environmental impact. Further, wastewater from synthetic Indigo production can contain heavy metals and chlorinated compounds, requiring extensive treatment to meet environmental safety standards. Although recent advances in green chemistry aim to reduce the toxicity of Indigo production by developing alternative pathways that avoid some of the more hazardous intermediates, these methods have yet to replace traditional processes at a large scale, primarily due to cost and the established infrastructure for classic synthetic Indigo production.

Thus, there is a need in the art for low environmental-impact dyes having consistent color hues and good color fastness when applied on fabric. Further, there is a need to select a particular color hue based on the dye creation process. The present invention addresses this need.

The present invention further provides techniques for enhancing the production of Indirubin. Indirubin is a bis-indole anti-tumor drug, which has an inhibitory effect on a variety of transplanted animal tumors, including the destruction of leukemia cells and is therefore an important material with separate utility.

### SUMMARY OF THE INVENTION

The present invention provides a biosynthesized Indigo dyestuff using L-tryptophan as starting materials and having at least 30% of Indigotin content, 1.5 times higher compared to plant-extracted Indigo. The overall conversion from L-tryptophan can reach up to 18.9% during biosynthesis. The Indigo dyestuff disclosed in the present invention can be harvested in less than a week, compared to 1 year for harvest from plant-extracted Indigo, resulting in shorter production times and higher yields. Indirubin, a byproduct of Indigo, can impart unique hues to Indigo dye. The present invention also discloses a composition providing a higher ratio of Indirubin to Indigotin in the Indigo dye by careful control of the biosynthesis environment.

In a first aspect the present invention provides a biosynthetic method for producing an Indigo dyestuff including Indigotin and Indirubin, includes generating tryptophanase (TRP) and flavin-containing monooxygenase (FMO) from a genetically-modified organism having a vector coding for tryptophanase and flavin-containing monooxygenase; catalyzing the conversion of L-tryptophan to Indigo dyestuff by the generated TRP and FMO in the presence of one or more bioactive additives selected from the cysteine, 2-oxindole, or 2-indoxyl to form a dyestuff having a ratio of Indigotin to Indirubin by weight between 1:0.1 to 1:4. The dyestuff has a color hue defined by CIE XYZ color space with X: 0.1920 to 0.2481, Y: 0.2537 to 0.2019, Z: 0.5543 to 0.5501.

In a further aspect, the present invention provides a composition for the biosynthesis of Indigo dyestuff including one or more of Indigotin and Indirubin. The composition includes a genetically engineered microorganism coding for expression of tryptophanase (TRP) and flavin-containing monooxygenase (FMO) along with 0 to 1.33 g/L (0 to 10 mM) of bioactive additives. The bioactive additives are one or more of cysteine, 2-oxindole, or 2-indoxyl. The composition is capable of regulating the biosynthesis of indigotin at 2 to 18.9% yield from L-tryptophan and Indirubin at 3 to 7.65% yield from L-tryptophan such that the ratio of Indigotin to Indirubin by weight is between 1:0.1 to 1:4 and the indigo dyestuff has a color hue defined by CIE XYZ color space with X: 0.1920 to 0.2481, Y: 0.2537 to 0.2019, Z: 0.5543 to 0.5501.

### BRIEF DESCRIPTION OF THE DRAWINGS

**FIG. 1** schematically depicts a pathway for the formation of Indigotin and Indirubin from tryptophan precursors.
**FIG. 2** schematically depicts a further pathway for the formation of Indirubin in the presence of cysteine.
**FIG. 3** is a graph showing the ratio of Indirubin to Indigotin compared to cysteine concentration.
**FIG. 4** is a graph showing the ratio of Indirubin to Indigotin compared to 2-indoxyl concentration in the presence of 0.030 g/L (0.25 mM) cysteine.
**FIG. 5** is a graph showing the ratio of Indirubin to Indigotin compared to cysteine concentration in the presence of 0.40 g/L (3 mM) 2-indoxyl.
**FIG. 6** is a schematic depiction of seed formation on textile fibers followed by dye formation on textile fibers.
**FIG. 7** is a graph showing the ratio of Indirubin to Indigotin compared to cysteine concentration used for dyeing wool fibers.
**FIG. 8** shows dye color based on CIE XYZ color space.

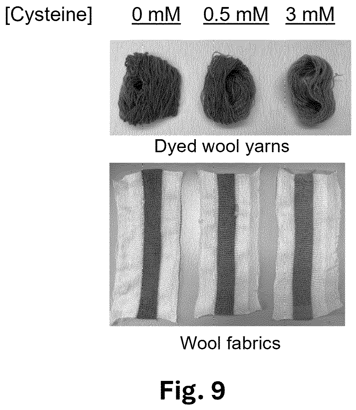

**FIG. 9** shows dyed wool yarns and fabrics according to cysteine concentration.

### DETAILED DESCRIPTION

The present invention provides biosynthesis of Indigo dyestuff, particularly with a controlled ratio of Indigotin (blue) and Indirubin (red). Traditionally, Indigo dye's color comes predominantly from blue Indigotin, but introducing a significant Indirubin fraction shifts the hue towards red-purple, which can be valuable for achieving unique color profiles. By being able to select a specific dye hue by tuning dye production conditions, custom dye production is facilitated.

### 1. Overview of Indigo dyestuff biosynthesis

Biosynthesis of Indigo dyestuffs relies on genetically-engineered host materials that provide enzymes for conversion of a selected raw material to a color-selected Indigo dyestuff. Indigo dyestuff, as used herein, is a dyestuff comprising Indigotin and Indirubin at a selected mass ratio. An exemplary starting raw material is L-tryptophan. This amino acid is cost-effective and can be produced by microbial fermentation from glucose or glycerol. L-tryptophan, acts as the primary precursor for indole, an intermediate needed for Indigo and Indirubin production. The enzyme, tryptophanase (TRP), catalyzes the conversion of L-tryptophan into indole. This reaction is a critical first step because indole serves as the key substrate for further conversion to indoxyl intermediates.

Flavin-containing monooxygenase (FMO) oxidizes indole to form 2-indoxyl and 3-indoxyl. The enzyme catalyzes selective hydroxylation at different positions on the indole ring, generating intermediates that are precursors to Indigotin and Indirubin.

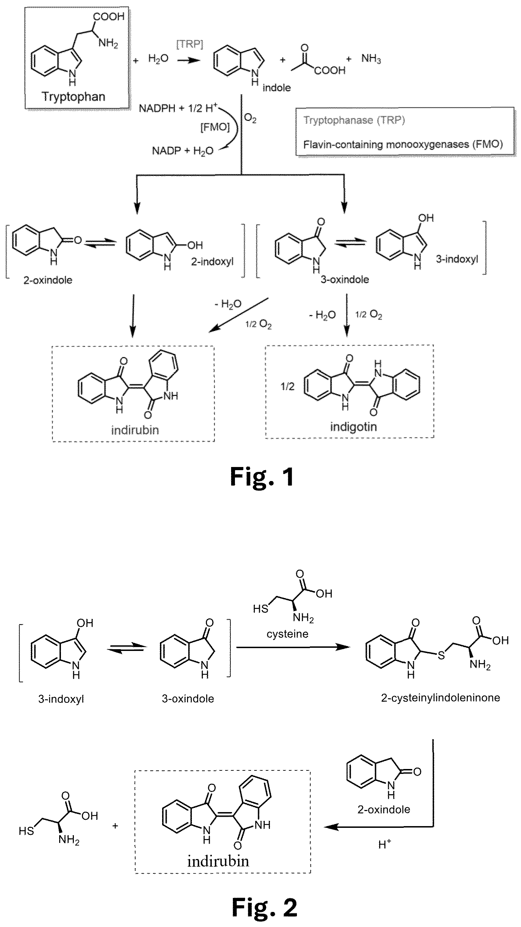

In the presence of oxygen, 3-indoxyl can undergo self-dimerization, producing Indigotin (blue Indigo). A reaction between 2-indoxyl and 3-indoxyl yields Indirubin. An overview of this reaction process is depicted in FIG. 1.

### 2. Microbial hosts for expression of enzymes

Several microorganisms may be used as hosts for enzyme production for Indigotin and Indirubin biosynthesis, each offering specific advantages in terms of enzyme compatibility, ease of genetic manipulation, and metabolic efficiency.

### a. Escherichia coli (E. coli)

*E. coli* is the most widely used microbial host for Indigo biosynthesis due to its well-characterized genetics, ease of manipulation, and rapid growth. It can be engineered to express high levels of TRP and FMO, making it efficient for catalyzing the conversion of L-tryptophan to indole, indoxyl, and subsequently Indigotin or Indirubin.

### b. Pseudomonas putida

Pseudomonas putida has a robust metabolic network that is tolerant to aromatic compounds, which makes it a good candidate for synthesizing and accumulating indole and indoxyl intermediates.

Non-bacteria hosts may also be used such as yeast.

A plasmid such as TRP-FMO pET29b includes the genes encoding TRP and FMO, enabling the microbial host to produce both enzymes and drive the production of indole and oxindole intermediates from L-tryptophan.

### 3. Environmental Control to Tune Ratio of Indigotin to Indirubin

As the present invention provides a controlled ratio of Indigotin to Indirubin, various factors and combination of factors that impact the production of Indirubin were determined in order to produce the desired ratios, as discussed in detail in the Examples below. As seen in FIG. 2, the formation of Indirubin can be promoted though addition of cysteine as a bioactive additive. Cysteine is converted to cysteinylindolenionone from indoxyl and oxindole, driving a pathway towards Indirubin rather than Indigotin. Cysteine also acts as a protecting group, helping preserve indoxyl's reactivity and availability for Indirubin formation.

2-indoxyl and 2-oxindole may be directly added as Indirubin precursors, to drive the reaction towards Indirubin formation. This additive approach can supplement the natural metabolic flow and create a higher local concentration of Indirubin precursors, enhancing the probability of Indirubin formation over Indigotin.

Other bioactive molecules such as 2-indoxyl and 2-oxindole at varying amounts (0.067 to 1.33 g/L) may be used to drive the reaction environment towards Indirubin formation. Indoxyl intermediates are oxygen-sensitive, and the presence of oxygen strongly influences whether they combine to form Indigotin or Indirubin.

### 4. Color Selection

The present invention provides biosynthesis of Indigotin to Indirubin by weight of between 1:0.1 to 1:4. The selected ratios of the present invention create a distinctive color that combines deep blue with purplish-red tones, resulting in a unique hue that is both visually appealing and versatile for modern textiles. This blend achieves a "softened blue" or "violet-blue" shade, which offers subtle complexity over the traditional deep blue of pure Indigo. The mix of Indigotin's deep blue with Indirubin's red-purple introduces a more nuanced color than pure Indigotin. This mix achieves a more complex shade. This hue differentiates itself from the more commonly used Indigo. For denims, it can resemble the softer, vintage look of naturally worn denim or aged textiles. This hue is especially appealing in clothing styles that seek to capture a weathered or "well-loved" appearance. For silk and wool, the Indigotin and Indirubin dyes can create different, subtle shades.

The selected color may be expressed in terms of CIE XYZ color space. CIE XYZ color space is one of the first color spaces to be defined mathematically, and was established in 1931 by the International Commission on Illumination (CIE). CIE XYZ uses three coordinates, X, Y and Z, which correspond to red, green, and blue color perception by the human eye. The use of CIE XYZ color space provides a standardized way of representing colors in a three-dimensional space, making it ideal for comparing and modifying colors produced through biosynthetic processes.

### 5. Direct Dyeing of Yarn or Fabric

Dyeing fabric or yarn directly in the culture medium used for biosynthesizing Indigotin and Indirubin may be used on the present invention. Since Indigo and Indirubin are produced in situ in the culture medium, dye molecules are in close contact with the textile substrate, which may improve dye uptake and adherence, especially for natural fibers like cotton and wool. The continuous exposure of the fabric or yarn to freshly synthesized dye in the medium may promote consistent coloration and deeper penetration into the fibers, potentially resulting in a more vivid and long-lasting color. By eliminating the need to extract and purify the dye before application, the total number of processing steps may be reduced, saving time and labor. This method bypasses the often energy-intensive extraction and solubilization steps traditionally needed with Indigo dye. Because biosynthesized Indigo dyestuff is present as indoxyl intermediates in the culture medium, it might adhere more readily without the need for additional chemicals to solubilize it, minimizing the environmental impact and complexity of the dyeing process.

If the dyeing process can occur directly in the culture medium, it may reduce the need for extensive water rinsing and chemical waste treatment, which is typically required when transitioning the dye from production to application. Since dyeing directly in culture medium could eliminate heat treatments often used to apply Indigo, there may be energy savings, especially if this process occurs at ambient temperatures.

### EXAMPLES

A genetically engineered microorganism is constructed in order to create enzymes for converting L-tryptophan into Indigo through the various transformations described below.

An *E. coli* is selected as a host microorganism. A plasmid is inserted into the *E. coli* having DNA coding for tryptophanase (TRP) and flavin-containing monooxygenase (FMO). The DNA construct in the 5' to 3' direction of transcription includes (i) a promoter that functions in the organism; (ii) a first transcribable nucleic acid sequence encoding a tryptophanase (TRP) according to SEQID 1, or a nucleotide sequence at least 90% identical thereto and capable of catalyzing conversion of L-tryptophan into indole; and (2) a second transcribable nucleic acid sequence encoding a flavin-containing oxygenase (FMO) according to SEQID 2, or a nucleotide sequence at least 90% identical thereto and encoding a polypeptide catalyzing formation of 3-indoxyl or 2-indoxyl from indole; and (iii) a transcriptional termination sequence. TABLE 1 provides the sequence listing of SEQID 1 and SEQID 2.

In certain embodiments, a TRP-FMO pET29b plasmid is used. The TRP-FMO pET29b plasmid contains T7 promotor, lactose operon, TRP and FMO genes, and the kanamycin resistance gene. T7 promotor is a sequence that can be identified by the T7 RNA polymerase for transcription of the plasmid. The lactose operon allows the transcription of the genes to form the recombinant protein regulated by the presence of lactose. The TRP catalyzes the conversion of L-tryptophan to intermediate compound, indole. Then, FMO continues to catalyze the conversion of indole to 2-oxindole and 3-oxindole. Kanamycin resistance gene is used for selection of bacteria containing the plasmid.

In some embodiments, a growing medium includes the bioactive additives in the growing medium including cysteine, 2-indoxyl and 2-oxindole for tuning the ratio between Indigotin and Indirubin. 0.030 to 0.48 g/L (0.25 to 4 mM) cysteine can tune the ratio of Indirubin to Indigotin from 1:0.1 to 1:4, where the CIE range is X: 0.1920-0.2481, Y: 0.2537-0.2019, Z: 0.5543-0.5501 in D65 light source.

In certain embodiments, wool yarns are immersed in the microorganism growing solution for in situ dying. Growing medium containing different concentrations (0 to 0.36 g/L equivalent to 0 to 3 mM) of cysteine yield ratios between Indigotin and Indirubin of 1:1 to 1:16 on the wool yarns.

### General procedure of production of Indigo dyestuff

The following procedure describes co-expression of TRP and FMO in bacteria. TRP-FMO pET29b plasmid was transformed into NEB^{®} 10-beta competent *E. coli.* 1 to 5 µL of TRP-FMO pET29b plasmid (50 pg to 100 ng) was transfected into 50 µL of competent cells. A mutant *E. coli* strain which is purchased from TWIST Bioscience is used to host the TRP-FMO pET29b. TRP (SEQID 1) and FMO (SEQID 2) are employed in the Examples.

The bacteria mixture was placed on ice for 30 minutes then heat shocked at 42°C for 30 seconds followed by placing on ice for 5 minutes. It was then shaken at 250 rpm 37°C for 60 min. The mixture was diluted and spread onto screening plate containing lactose, kanamycin and L-tryptophan and incubated overnight. A single colony grown on Luria-Bertani (LB) agar plate containing 50 µg/mL kanamycin was used to inoculate 10 mL LB broth containing 50 µg/mL kanamycin, and was grown and shaken overnight at 30°C at 250 rpm. When the optical density at 600 nm (OD600) of the resulting cell culture reached 0.8 (as measured by a Jenway^{™} 72 Series Genova Bio Spectrophotometer at 600 nm), 1 mL of this cell culture was added to 100 mL fermentation medium (20 g/L LB broth, 50 µg/mL kanamycin, 1.7 g/L lactose and 2 g/L L-tryptophan). The ingredients have been optimized for Indigo dyestuff production. The culture was further incubated at 30°C with shaking at 180 rpm for 72 h. After 72 h, water-insoluble blue precipitate was observed in the broth.

The suspension was collected and centrifuged at 8000 rpm for 10 min. The sediment was washed with deionized water 2 times and allowed to be freeze dried. Crude solid was defined as the solid collected after freeze drying. The weight of the crude solid was measured. The crude solid was dissolved in DMSO, filtered with 0.45 µm PTFE filter and analyzed with HPLC. The signals of Indirubin and Indigotin were observed with a photodiode array detector with the wavelength 500 nm and 620 nm respectively.

Examples 1 to 7 describe the production of Indigo dyestuff with different concentrations of cysteine in the medium.

The formation of Indirubin can be promoted though the addition of cysteine. Cysteine is converted to 2-cysteinylindolenionone from indoxyl and oxindole, driving a pathway towards Indirubin rather than Indigotin. Cysteine also acts as a protecting group, helping preserve indoxyl's reactivity and availability for Indirubin formation.

TABLE 2 tabulates the results of Examples 1 to 7 with different concentrations of cysteine, Isolated Yield of Indigo dyestuff, Indirubin Content, Indigotin Content, Conversion to Indirubin and Indigotin from L-tryptophan, and the ratio between Indigotin to Indirubin.

Isolated Yield of Indigo dyestuff was defined as the mass of crude solid that can produce with 1 L medium.

Indirubin Content and Indigotin Content were the percentage of the compounds of interest in the crude solid. It was obtained by dividing the concentration of each Indigo dyestuff by the concentration of the crude solution dissolving in DMSO (200 ppm).

Conversion (in percentage) to Indirubin and Indigotin from L-tryptophan were calculated by multiplying the Indirubin Content or Indigotin Content with the Isolated Yield of Indigo dyestuff and divided by the theoretical yield of Indigo dyestuff from the L-tryptophan.

The ratio between Indigotin to Indirubin was calculated by the concentrations of Indigotin and Indirubin deduced from the HPLC analysis.

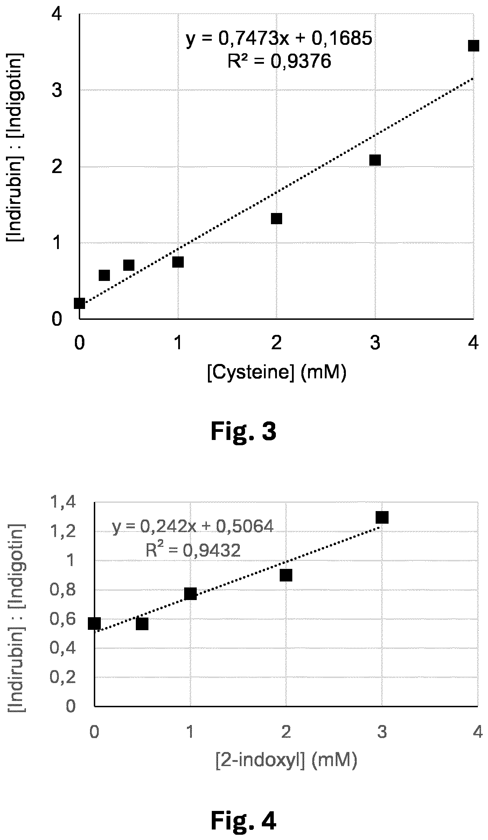

FIG. 3 shows the increasing of Cysteine enhanced the production of Indirubin over Indigotin. The isolated theoretical yield of Indirubin increases from 3.37 to 7.65%. The isolated theoretical yield of Indigotin decreases from 18.9 to 2.14%.

2-indoxyl and 2-oxindole may be directly added as Indirubin precursors, to drive the reaction towards Indirubin formation. This additive approach can supplement the natural metabolic flow and create a higher local concentration of Indirubin precursors, enhancing the probability of Indirubin formation over Indigotin.

Isatin is also a precursor in the formation of Indirubin via oxidative coupling. Addition of isatin may drive the reaction towards Indirubin formation.

Examples 8, 9 and Comparative Example 10 describes the production of Indigo dyestuff in the presence of 10 mM of 2-indoxyl (1.33 g/L), 2-oxindole (1.33 g/L) or isatin (1.47 g/L) in the medium.

TABLE 3 tabulates the isolated Yield of Indigo dyestuff, Indirubin Content, Indigotin Content, Isolated Yield of Indirubin and indigotin, and the ratio between Indigotin to Indirubin from Examples 8, 9 and Comparative Example 10.

Only Indirubin is detected in Examples 8 and 9 due to high concentration of the bioactive additives, but low in isolated yield. The high concentration of bioactive additives may act as growth inhibitor of *E. coli.*

FIG. 4 shows increasing 2-indoxyl concentration (0, 0.067, 0.13, 0.27 and 0.40 g/L, equivalent to 0, 0.5, 1, 2 and 3 mM) at fixed cysteine concentration (0.030 g/L or 0.25 mM) enhanced the production of Indirubin over Indigotin, but not at the same increment when increasing cysteine alone.

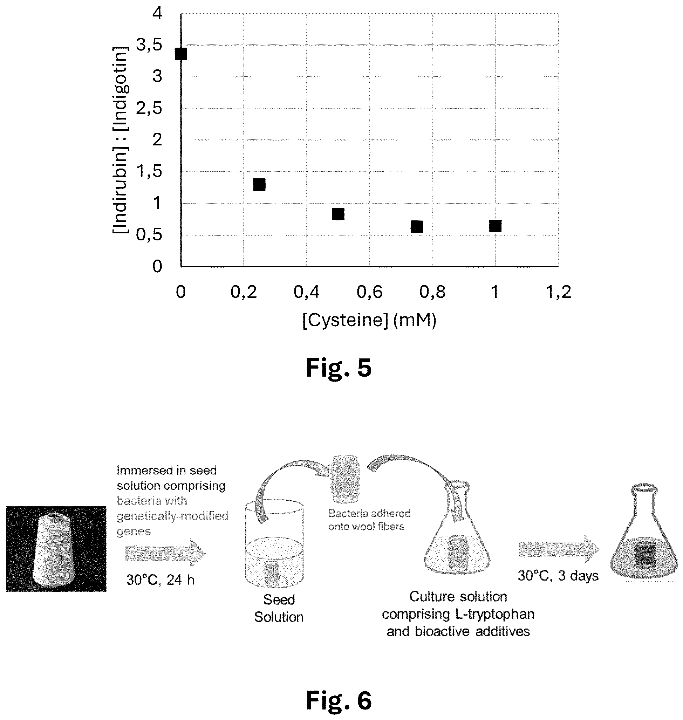

FIG. 5 shows increase of cysteine concentration (0, 0.030, 0.061, 0.091 and 0.12 g/L, equivalent to 0, 0.25, 0.5, 0.75 and 1 mM) at fixed 2-indoxyl concentration (0.40 g/L or 3 mM) decreases production of Indirubin over Indigotin.

The effect of 2-indoxyl to enhance indirubin production is not prominent and might show inhibition effect. The use of cysteine alone is preferred.

Examples 11 to 13 describe production and in situ dyeing of indigo dyestuff directly wool yarn in medium containing different concentrations of cysteine.

The dyeing process was illustrated in FIG. 6. In the first step, a 5 mL seed solution was prepared by using 10 µL cryopreserved transformed *E. coli* using fermentation medium comprising 20 g/L LB broth, 2 g/L L-tryptophan, 1.71 g/L lactose, and 50 mg/L Kanamycin stock, and thereafter activated at 30° C for 12 h. Wool yarns (1 g) which were previously autoclaved at 121°C, were immersed into this seed solution, and placed in an incubator at 30°C for 24 h. This process allows transformed *E. coli* to be absorbed on wool yarns.

In the second step, these wool yarns were transferred to a freshly prepared fermentation medium (100 mL) with different cysteine concentrations (0, 0.061 and 0.36 g/L, equivalent to 0, 0.5 and 3 mM). It was then incubated at 30°C for 72 h with 180 rpm agitation. The resulting Indigo dyestuff that was formed affixed on wool yarns. They were sterilized at 60°C for 30 min, and were washed with distilled water several times, then air dried. The medium and the wool yarns were then extracted with DMSO for quantification of Indigo dyestuff. The resulting solution was then filtered with 0.45 µm PTFE filter prior to HPLC analysis.

Examples 11 to 13 summarize the Indigo dyestuff content in the wool yarns. TABLE 4 tabulates the results of Examples 11 to 13 with different concentrations of cysteine (0, 0.061 and 0.36 g/L, equivalent to 0, 0.5 and 3 mM), and the ratio between Indigotin to Indirubin.

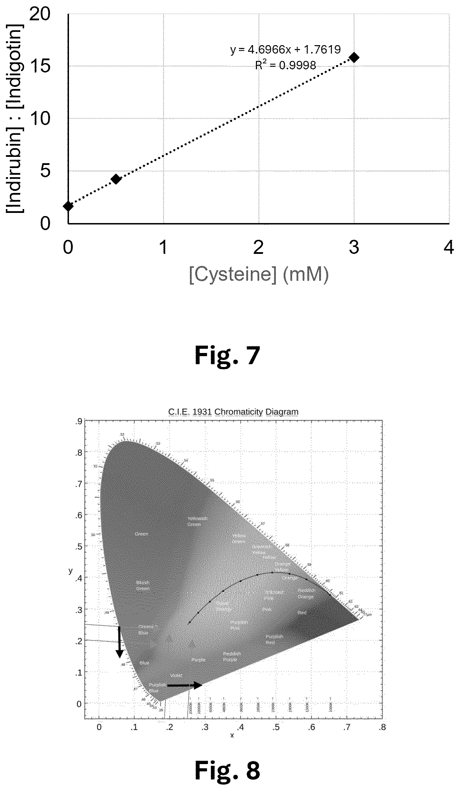

FIG. 7 shows that the increase of cysteine concentration enhanced the production of Indirubin over Indigotin in the wool yarns. The overall ratio of Indirubin content over Indigotin is higher in the wool yarn than in the medium used for incubation. The highest ratio between Indigotin to Indirubin was 1:15.8. This ratio is much higher compared to Indigo dyestuff produced in culture medium in the absence of wool yarns. Color fastness to rub (ISO 105 X-12) of the resulting wool fabrics dyed with the Indigo dyestuff using methods disclosed in the invention achieved Grade 4 to 5.

The Indigo dyestuff was further quantified using CIE XYZ color space.

TABLE 5 tabulates the CIE XYZ values of Examples 1 to 7 with different concentrations of cysteine (0, 0.030, 0.061, 0.12, 0.24, 0.36 and 0.48 g/L, equivalent to 0, 0.25, 0.5, 1, 2, 3 and 4 mM). The Indigo dyestuff was dissolved in DMSO at fixed concentration (200 ppm).

At a given cysteine concentration, the ratio of Indirubin to Indigotin can be tuned from 1:0.1 to 1:4, where the CIE values are X: 0.1920 to 0.2481, Y: 0.2537 to 0.2019, Z: 0.5543 to 0.5501 under D65 light source.

**TABLE 1**

| Name | Organism | Feature | Sequence | Remarks |
|---|---|---|---|---|
| SEQ ID NO:1 | Escherichia coli strain ECJHY21-04 | Tryptophanase | ATGGAGAACTTTAAGCATTTACCTGAGCCATTCCG GATCCGTGTTATTGAGCCAGTTAAACGGACAACC CGAGCATATCGTGAAGAAGCGATCATAAAATCAG GTATGAATCCTTTTTTGCTGGATAGCGAAGATGTT TTTATTGACTTGCTGACAGATTCCGGTACAGGAGC CGTTACGCAAAGTATGCAGGCTGCGATGATGAGA GGCGACGAGGCGTACAGCGGTTCTCGTTCGTACT ATGCCCTTGCAGAGAGTGTTAAAAATATTTTTGGT TATCAATACACCATACCGACGCACCAAGGACGCG GGGCAGAACAAATCTATATACCCGTGTTGATCAAA AAGCGCGAACAGGAAAAGGGACTCGATCGATCC AAAATGGTGGCCTTTTCCAATTATTTTTTTGACAC CACTCAAGGTCATTCCCAGATTAACGGCTGTACCG TGCGGAATGTATATATTAAAGAAGCATTCGATACT GGGGTGCGTTATGACTTTAAAGGCAATTTCGATCT GGAGGGTTTAGAACGCGGCATAGAAGAAGTTGG GCCAAATAATGTTCCTTACATCGTTGCAACCATTA CCTCAAATAGTGCGGGTGGCCAACCTGTTTCATTA GCGAACCTCAAAGCCATGTATTCCATTGCGAAAA AATATGATATTCCTGTGGTGATGGATTCCGCAAGA TTCGCGGAAAATGCGTATTTCATAAAACAGCGCG AGGCTGAATATAAAGACTGGACAATTGAACAAAT CACACGTGAAACGTATAAATATGCTGACATGCTTG CTATGAGCGCGAAAAAAGATGCGATGGTCCCTAT GGGGGGGCTTCTGTGTATGAAAGATGATTCGTTTT TTGACGTTTACACGGAGTGTCGTACGCTTTGTGTA GTACAAGAAGGCTTTCCAACATATGGCGGCCTCG AAGGGGGCGCAATGGAACGTCTTGCAGTTGGCCT TTATGATGGAATGAATTTAGATTGGCTGGCATACC GTATTGCCCAGGTCCAGTATCTGGTGGATGGCCTC GAAGAAATCGGCGTTGTGTGTCAGCAGGCCGGTG GTCACGCCGCATTTGTTGATGCCGGCAAACTGCTT CCGCACATCCCGGCAGACCAATTTCCTGCGCAGG CACTGGCGTGCGAACTGTATAAGGTGGCGGGTAT | Synthetic Construct, GenBank: MCK2276239. 1 |
| an-original | | | | |
| | | | CCGCGCCGTGGAGATAGGCAGTTTTCTTCTTGGG CGTGATCCGAAAACCGGGAAACAACTGCCATGCC CAGCGGAACTGCTGCGCCTCACGATACCGCGTGC GACATATACCCAAACCCATATGGATTTCATCATTGA AGCGTTTAAACATGTGAAAGAAAATGCCGCGAAC ATTAAAGGCTTAACTTTTACCTATGAGCCAAAAGT TCTGCGGCACTTTACAGCAAAATTGAAAGAAGTT TAAC | |
| SEQ ID NO:2 | Methylophag a sp. SK1 | Flavin-containing monooxygenas e | ATGGCAACTCGAATTGCAATTTTAGGTGCTGGGCC AAGCGGCATGGCGCAGCTGCGCGCGTTCCAGTCT GCCCAAGAAAAAGGCGCAGAAATTCCTGAGTTG GTGTGCTTCGAAAAACAGGCCGACTGGGGTGGC CAATGGAATTATACATGGAGAACAGGGTTGGACG AAAACGGTGAGCCTGTTCACTCTTCGATGTACCG TTACCTGTGGTCTAACGGACCAAAAGAGTGCCTC GAATTTGCCGACTACACCTTTGATGAGCATTTCGG CAAACCTATTGCATCGTATCCACCGAGAGAAGTTT TATGGGACTATATAAAAGGTCGCGTAGAGAAGGC AGGCGTTCGGAAATACATTAGATTTAATACTGCGG TGCGCCACGTCGAATTCAACGAAGATTCCCAAAC ATTTACGGTGACAGTACAGGATCATACTACAGACA CCATTTATTCAGAGGAATTTGATTATGTGGTCTGCT GTACCGGCCACTTTAGCACGCCTTACGTGCCTGA ATTTGAAGGTTTTGAGAAATTTGGTGGTCGAATTC TGCATGCGCATGATTTCCGGGATGCTTTAGAGTTC AAAGATAAGACGGTACTGCTGGTTGGTTCATCCTA CTCTGCGGAAGATATAGGGTCACAGTGCTATAAGT ATGGGGCAAAAAAGCTGATTAGTTGTTACCGCAC CGCACCTATGGGATATAAATGGCCGGAAAACTGG GATGAGCGTCCTAATTTAGTTCGTGTAGATACGGA AAATGCATATTTCGCTGATGGCTCATCTGAAAAAG TGGACGCTATAATCCTCTGCACGGGTTATATTCATC ACTTTCCATTCCTGAACGACGACCTGCGCTTGGT GACAAACAATCGTCTGTGGCCGCTCAACCTGTAT AAAGGTGTGGTCTGGGAAGATAATCCTAAATTTTT TTATATAGGGATGCAGGATCAGTGGTATTCTTTTAA TATGTTTGACGCCCAGGCATGGTATGCGCGAGATG TTATTATGGGACGTCTCCCGCTTCCGAGTAAAGAG GAAATGAAAGCTGACTCAATGGCTTGGCGCGAAA AAGAACTGACTTTGGTCACGGCGGAAGAGATGTA TACTTATCAAGGCGATTACATCCAGAATCTTATTG ATATGACAGACTATCCGTCTTTTGATATTCCCGCAA CCAACAAAACCTTTTTGGAATGGAAACATCACAA AAAAGAAAATATTATGACGTTCCGTGATCATAGCT ACCGCAGCCTTATGACTGGCACAATGGCCCCTAA GCATCATACTCCCTGGATTGATGCCCTGGATGATT CTTTGGAAGCGTATCTCTCCGATAAATCGGAAATC CCAGTTGCCAAGGAGGCATAA | Synthetic Construct, GenBank: AF494423.2 |

**TABLE 2**

| | Example 1 | Example 2 | Example 3 | Example 4 | Example 5 | Example 6 | Example 7 |
|---|---|---|---|---|---|---|---|
| [cysteine] (mM)* | 0.0 | 0.25 | 0.5 | 1.0 | 2.0 | 3.0 | 4.0 |
| Isolated Yield of Indigo dyestuff (g/L) | 0.810 | 0.745 | 0.752 | 0.786 | 0.746 | 0.749 | 0.686 |
| Indirubin Content (%)** | 5.34 ± 0.17 | 6.35 ± 0.19 | 9.51 ± 0.21 | 5.17 ± 0.17 | 8.45 ± 0.21 | 11.7 ± 0.2 | 14.3 ± 0.2 |
| Indigotin Content (%)** | 30.0 ± 0.3 | 11.1 ± 0.3 | 13.5 ± 0.3 | 6.93 ± 0.20 | 6.43 ± 0.17 | 5.62 ± 0.19 | 4.00 ± 0.18 |
| Conversion to Indirubin from tryptophan (%) | 3.37 ± 0.11 | 3.68 ± 0.11 | 5.57 ± 0.13 | 3.16 ± 0.11 | 4.91 ± 0.12 | 6.84 ± 0.10 | 7.65 ± 0.10 |
| Conversion to Indigotin from tryptophan (%) | 18.9 ± 0.2 | 6.43 ± 0.17 | 7.90 ± 0.15 | 4.24 ± 0.12 | 3.73 ± 0.10 | 3.28 ± 0.11 | 2.14 ± 0.09 |
| [Indigotin] : [Indirubin] | 1: 0.18 | 1 : 0.57 | 1: 0.71 | 1: 0.75 | 1 : 1.31 | 1:2.09 | 1:3.58 |

| | | | | | | | |
|---|---|---|---|---|---|---|---|
| *Fermentation medium comprising: [L-tryptophan] = 2 g/L; [Luria-Bertani (LB) broth] = 20 g/L; [Lactose] = 1.7 g/L; [Kanamycin]: 50 mg/L; Volume of fermentation medium = 100 mL **Based on HPLC analysis of isolated Indigo dyestuff Data presented as mean ± S.D. (n = 3) | | | | | | | |

**TABLE 3**

| | Example 8 | Example 9 | Comparative Example 10 |
|---|---|---|---|
| *Bioactive Additive | 2-indoxyl | 2-oxindole | isatin |
| Isolated Yield of Indigo dyestuff (g/L) | 0.114 | 0.124 | 0.128 |
| Indirubin Content (%)** | 1.00 | 1.08 | n.d. |
| Indigotin Content (%)** | n.d. | n.d. | n.d. |
| [Indigotin] : [Indirubin] | 0:1 | 0:1 | n.d. |

| | | | |
|---|---|---|---|
| [Bioactive additive] = 10 mM *Fermentation medium comprising: [L-tryptophan] = 2 g/L; [Luria-Bertani (LB) broth] = 20 g/L; [Lactose] = 1.7 g/L; [Kanamycin]: 50 mg/L; Volume of fermentation medium = 100 mL **Based on HPLC analysis of isolated Indigo dyestuff n.d.: not detectable | | | |

**TABLE 4**

| | Example 8 | Example 9 | Example 10 |
|---|---|---|---|
| [cysteine] (mM)* | 0 | 0.5 | 3 |
| [Indigotin] : [Indirubin] in wool yarn | 1 : 1.67 | 1: 4.22 | 1:15.8 |

| | | | |
|---|---|---|---|
| *Fermentation medium comprising: [ L-tryptophan] = 2 g/L; [Luria-Bertani (LB) broth] = 20 g/L; [Lactose] = 1.7 g/L; [Kanamycin]: 50 mg/L; Volume of fermentation medium = 100 mL | | | |

**TABLE 5**

| | Example 1 | Example 2 | Example 3 | Example 4 | Example 5 | Example 6 | Example 7 |
|---|---|---|---|---|---|---|---|
| [cysteine] (mM)* | 0.0 | 0.25 | 0.5 | 1.0 | 2.0 | 3.0 | 4.0 |
| CIE x | 0.1920 | 0.2020 | 0.2074 | 0.2264 | 0.2240 | 0.2347 | 0.2481 |
| CIE y | 0.2537 | 0.2330 | 0.2370 | 0.2569 | 0.2290 | 0.2111 | 0.2019 |
| CIE z | 0.5543 | 0.5650 | 0.5556 | 0.5167 | 0.5470 | 0.5543 | 0.5501 |

### 6. Industrial Applicability and Advantages

The biosynthesized Indigo-based dyestuffs of the present invention offer several distinct advantages over traditional synthetic Indigo, particularly in terms of sustainability, reproducibility, and environmental safety.

As described above, biosynthetic methods allow for precise control over the reaction pathways, which can lead to more consistent color from batch to batch. By regulating factors like enzyme expression, intermediate concentrations, and reaction conditions, manufacturers can achieve a reproducible Indigotin-to-Indirubin ratio, ensuring uniform color in every lot.

Precision in Color Tuning: With the ability to control Indirubin and Indigotin content, biosynthesis offers an unprecedented ability to customize shades. By measuring color in CIE XYZ values, manufacturers can standardize colors precisely, reducing the variability commonly seen in synthetic and plant-derived Indigo dyes.

Enhanced Dye Quality: While color fastness in Indigo is largely determined by its chemical structure, controlling dye purity and reducing impurities in biosynthetic processes can contribute to more consistent color fastness. This can result in dye that adheres more uniformly to fibers, which may provide improved wash and light fastness.

Better Control of Additives: Biosynthetic pathways can allow for the addition of stabilizers and modifiers at specific points in the metabolic process, potentially enhancing color retention without affecting the dye's natural properties.

Direct Production of Indoxyl Precursors: Traditional synthetic Indigo requires a chemical reduction step to make the dye water-soluble for textile application, which typically involves toxic reducing agents like sodium hydrosulfite. Biosynthesized Indigo, however, can be produced in the indoxyl form, which may simplify the dyeing process by reducing the need for additional chemicals.

Potential for Lower Temperature Processing: Biosynthetic dyes can be applied at lower temperatures, reducing the energy requirements of the dyeing process. This is particularly beneficial in industrial applications where energy use is a major cost factor.

Elimination of Harmful Reducing Agents: Synthetic Indigo production traditionally relies on toxic substances, including aniline and heavy metals. By contrast, biosynthetic Indigo production occurs in microbial cultures without the need for harsh chemicals, resulting in a cleaner production process with less hazardous waste.

Lower Environmental Impact: The absence of toxic precursors and reducing agents in biosynthetic Indigo production means fewer pollutants in wastewater. This is especially important as dye industry effluents can contain toxic compounds that affect aquatic ecosystems.

Microbial Production Using Renewable Feedstocks: Biosynthetic Indigo is produced using microorganisms, such as *E. coli,* or Saccharomyces cerevisiae, grown on renewable feedstocks like glucose. This contrasts with synthetic methods, which rely on petrochemicals and non-renewable resources.

Reduced Carbon Footprint: Since biosynthesis can operate under milder conditions and at lower temperatures, it requires less energy, reducing the overall carbon footprint of Indigo dye production.

Industrial-Scale Fermentation: Microbial fermentation systems are well-suited to large-scale operations. By growing engineered bacteria in fermentation tanks, Indigotin and Indirubin can be produced at industrial volumes. Similar biosynthetic approaches are already used commercially for compounds like insulin and various amino acids, so scaling this pathway is achievable with current biotechnology.

Fermentation Efficiency: High-density fermentation methods, optimized nutrient feeds, and controlled oxygen levels are key to efficiently producing high concentrations of Indigo precursors. High-throughput screening of engineered strains can improve pathway efficiency and yield.

Several embodiments of the present disclosure and features of details are briefly described above. The embodiments described in the present disclosure may be easily used as a basis for designing or modifying other processes and structures for realizing the same or similar objectives and/or obtaining the same or similar advantages introduced in the embodiments of the present disclosure. Such equivalent construction does not depart from the spirit and scope of the present disclosure, and various variations, replacements, and modifications can be made without departing from the spirit and scope of the present disclosure.

As used herein, terms "approximately", "basically", "substantially", and "about" are used for describing and explaining a small variation. When being used in combination with an event or circumstance, the term may refer to a case in which the event or circumstance occurs precisely, and a case in which the event or circumstance occurs approximately. As used herein with respect to a given value or range, the term "about" generally means in the range of ±10%, ±5%, ±1%, or ±0.5% of the given value or range. The range may be indicated herein as from one endpoint to another endpoint or between two endpoints. Unless otherwise specified, all the ranges disclosed in the present disclosure include endpoints. The term "substantially coplanar" may refer to two surfaces within a few micrometers (µm) positioned along the same plane, for example, within 10 µm, within 5 µm, within 1 µm, or within 0.5 µm located along the same plane. When reference is made to "substantially" the same numerical value or characteristic, the term may refer to a value within ±10%, ±5%, ±1%, or ±0.5% of the average of the values.

## Claims

1. A biosynthetic method for producing an Indigo dyestuff including Indigotin and Indirubin, comprising:
generating tryptophanase (TRP) and flavin-containing monooxygenase (FMO) from a genetically-modified organism having a vector coding for tryptophanase and flavin-containing monooxygenase;
catalyzing the conversion of L-tryptophan to Indigo dyestuff by the generated TRP and FMO in the presence of one or more bioactive additives selected from the group consisting of cysteine, 2-oxindole, and 2-indoxyl, to form a dyestuff having a ratio of Indigotin to Indirubin by weight between 1:0.1 to 1:4, the dyestuff having a color hue defined by CIE XYZ color space with X: 0.1920 to 0.2481, Y: 0.2537 to 0.2019, Z: 0.5543 to 0.5501.

2. The biosynthetic method of claim 1, wherein the bioactive additive includes cysteine in an amount of 0.03 to 0.48 g/L.

3. The biosynthetic method of claim 1, wherein the bioactive additive includes 2-oxindole in an amount of 1.33 g/L.

4. The biosynthetic method of claim 1, wherein the bioactive additive includes 2-indoxyl in an amount of 0.067 to 1.33 g/L.

5. The biosynthetic method of claim 1, wherein the vector includes a first transcribable nucleic acid sequence encoding a tryptophanase according to SEQID 1, or a nucleotide sequence at least 90% identical thereto, and a second transcribable nucleic acid sequence encoding a flavin-containing oxygenase (FMO) according to SEQID 2, or a nucleotide sequence at least 90% identical thereto.

6. The biosynthetic method of claim 1, wherein the vector is a TRP-FMO pET29b plasmid.

7. An Indigo dyestuff made by the process of claim 1.

8. A composition for the biosynthesis of indigo dyestuff comprises one or more of Indigotin and Indirubin comprising:
a genetically-engineered microorganism coding for expression of tryptophanase (TRP) and flavin-containing monooxygenase (FMO);
0.03 to 1.33 g/L of bioactive additives;
wherein the bioactive additives comprise one or more of cysteine, 2-oxindole, or 2-indoxyl;
wherein the said composition is capable of regulating the biosynthesis of indigotin at 2 to 18.9% yield from L-tryptophan and Indirubin at 3 to 7.65% yield from L-tryptophan;
wherein the ratio of Indigotin to Indirubin by weight is between 1:0.1 to 1:4;
wherein the indigo dyestuff has a color hue defined by CIE XYZ color space with X: 0.1920 to 0.2481, Y: 0.2537 to 0.2019, Z: 0.5543 to 0.5501.

9. The composition of claim 8, wherein the bioactive additive includes cysteine in an amount of 0.03 to 0.48 g/L.

10. The composition of claim 8, wherein the bioactive additive includes 2-oxindole in an amount of 1.33 g/L.

11. The composition of claim 8, wherein the bioactive additive includes 2-indoxyl in an amount of 0.067 to 1.33 g/L.

12. The composition of claim 8, wherein the vector includes a first transcribable nucleic acid sequence encoding a tryptophanase according to SEQID 1, or a nucleotide sequence at least 90% identical thereto, and a second transcribable nucleic acid sequence encoding a flavin-containing oxygenase (FMO) according to SEQID 2, or a nucleotide sequence at least 90% identical thereto.

13. The composition of claim 8, wherein the vector is a TRP-FMO pET29b plasmid.

14. A biosynthetic method for in situ fabric dyeing by an Indigo dyestuff including Indigotin and Indirubin, comprising:
generating tryptophanase (TRP) and flavin-containing monooxygenase (FMO) from a genetically-modified organism having a vector coding for tryptophanase and flavin-containing monooxygenase in the presence of a fabric or yarn to be dyed;
catalyzing the conversion of L-tryptophan to Indigo dyestuff by the generated TRP and FMO in the presence of a fabric or yarn to be dyed and in the presence of one or more bioactive additives selected from the group consisting of cysteine, 2-oxindole, and 2-indoxyl, to form a dyed fabric or yarn having a ratio of Indigotin to Indirubin by weight between 1:1 to 1:16, the dyestuff having a color hue defined by CIE XYZ color space with X: 0.1920 to 0.2481, Y: 0.2537 to 0.2019, Z: 0.5543 to 0.5501.

15. The biosynthetic method for in situ fabric dyeing of claim 14, wherein the generating TRP in the presence of a fabric or yarn to be dyed is from a seed solution of the genetically-modified organism having a vector coding for tryptophanase and flavin-containing monooxygenase includes absorbing the genetically-modified organism on the wool fabric or yarn followed by conversion of L-tryptophan to Indigo dyestuff catalyzed by the generated TRP and FMO.
